# EUROPÄISCHE PATENTSCHRIFT

(11) **EP 2 366 410 B1**
(45) Veröffentlichungstag und Bekanntmachung des Hinweises auf die Patenterteilung: **06.08.2014**
(21) Anmeldenummer: 11001839.7
(22) Anmeldetag: 04.03.2011
(51) Int. Cl.: A61L 2/07, B01J 3/04

(54) **Ventil für Fluid Ein- und Auslass**
Valve for fluid inlet and outlet
Valve pour entrée et sortie de fluide

(30) Priorität: 05.03.2010 DE 202010003204 U
(43) Veröffentlichungstag der Anmeldung: 21.09.2011
(73) Patentinhaber: WAGNER GMBH FABRIK FÜR MEDIZINISCHE GERÄTE, 80634 München (DE)
(72) Erfinder: Wagner, Peter, 82319 Starnberg (DE)
(74) Vertreter: Feldkamp, Rainer

(56) Entgegenhaltungen:
- DE-C1- 19 913 417
- DE-U1- 8 711 702
- DE-U1- 8 900 027
- US-A- 4 748 003

## Beschreibung

Die Erfindung betrifft ein Ventil für einen in der Behälterwand eines für die Dampf-Autoklavierung bestimmten Behälters angeordneten Fluid-Ein-/Auslass. Derartige Ventile steuern Strömungen in einen Behälter, die abhängig von einem bestimmten Parameter (Druck und/oder Temperatur) einströmen und unter vorbestimmten anderen Umgebungsbedingungen ausströmen können, wobei insbesondere dann, wenn die vorbestimmten Umgebungsbedingungen nicht vorliegen, ein dauerhafter dichter Abschluss gewährleistet wird.

Die US-PS 4 748 003 zeigt einen aus Bodenteil und Deckel bestehenden Sterilisierbehälter, der im vertleften Teil seines Bodens und erhöhten Teil seines Deckels je ein derartiges Ventil zeigt. Durch das im Deckel angeordnete Ventil strömt heißer Dampf In das Innere des Behälters, und aus dem unteren Ventil strömt kalte Luft aus, wenn beide Ventile offen sind. Druckabhängige Elemente in Form von Faltenbalgen sind so eingestellt, dass sie die Ventile schließen, wenn der Außendruck gleich dem atmosphärischen Druck ist, und die schnell voll öffnen, wenn der Druck anzusteigen beginnt und Dampf zugeführt wird, um den Sterilisationszyklus einzuleiten. Wenn der Außendruck am Ende des Zyklus auf atmosphärischen Druck zurückkehrt, schließen die druckbetätigten Ventile um den Sterilisierbehälter zur Entnahme aus dem Autoklaven abzudichten. Der Ventilsitz wird von einem elastomeren Ring im Deckel und Boden gebildet, gegen den ein Ventilkörper anpressbar ist, dessen Durchtrittsloch durch einen Faltenbalg druckabhängig absperrbar ist. Die Ventile sind im Deckel und Boden fest eingebaut.

Einen anderen Sterilisierbehälter mit fest eingebauten Ventilen zeigt auch die deutsche Gebrauchsmusterschrift 8900027. Hier ist ein Faltenbalg vorgesehen, der einerseits an der Behälterwand und andererseits an einer Abdeckscheibe abgestützt ist, die dadurch gegen eine Filterscheibe und gegen eine Stirnwand entgegen einer Schraubenfeder verschoben wird.

Die Gebrauchsmusterschrift 8711702 zeigt ein Ventil für einen Sterilisierbehälter, das während der gesamten Vakuumphase und der Sterilisierphase und der Vakuumtrocknungsphase offen hält, und das während der Belüftungsphase des Sterilisierbehälters schließt. Dies geschieht dadurch, dass ein Ventilkörper durch eine Druckkapsel gegen die Kraft einer Schließfeder offen gehalten wird. Diese Druckkapsel ist während des Sterllisiervorganges über ein offenes zweites Ventil und ein Rückschlagventil mit dem Behälterinneren verbunden und wird durch eine Ventilkugel erst während der Belüftungsphase geschlossen, so dass dann infolge der entstehenden erheblichen Druckdifferenz die Kapsel zusammengedrückt wird und der Ventilkörper auf den Ventilsitzen aufsitzen kann.

Die DE 19913417 C1 zeigt einen Sterilisierbehälter mit einer Ventilanordnung, die durch Schnappscheiben gesteuert wird, wobei die Ventilanordnung einen dem Strömungsdruck ausgesetzten Ventilkörper aufweist, der durch einen Anschlag gegen ein Schließen gesperrt ist und der Anschlag vor und während der letzten Belüftungsphase durch die auftretende Druckdifferenz über die Schnappscheiben unwirksam gemacht wird.

Der Erfindung liegt die Aufgabe zugrunde, ein Ventil für einen Fluld-Eln-/Auslass eines Sterilisierbehälters zu schaffen, das zuverlässig in Abhängigkeit von bestimmten Parametern öffnet und schließt und den Einlass geöffnet oder den Auslass geschlossen hält, wie dies durch die Umgebungsbedingungen erforderlich ist, wobei die Einzelteile der Ventilanordnung einfach austauschbar ist.

Gelöst wird die gestellte Aufgabe durch die Gesamtheit der Im Patentanspruch 1 angegebenen Merkmale.

Der erfindungsgemäß vorgesehene Faltenbalg steuert druckabhängig den Fluid-Einbeziehungsweise Auslass, wobei dieses druckabhängig steuernde Stellgliedeinfach durch Aufrasten ersetzbar angeordnet ist und mit seinen beidseitig identisch ausgebildeten Deckeln wahlweise einen Rastkörper zur Festlegung an einem Behälterteil oder ein Verschlussglied bildet. Diese Ausbildung ermöglicht es, den das Stellglied mit den beiden Absperrorganen bildenden Faltenbalg auf einfache Weise zu entnehmen und durch Umkehrung des Faltenbalges ein neues Verschlussorgan zum Einsatz zu bringen.

Das erfindungsgemäße Ventil gewährleistet, dass so lange vor, während oder nach der Autoklavierung der in der abgedichteten Druckkammer herrschende Druck höher oder gleich dem das Stellglied umgebenden Außendruck ist, sich der zylindrische Faltenbalg des Stellgliedes spreizt und dadurch das Absperrorgan schließt, und dass dann, wenn während der Autoklavierung der das Stellglied umgebende Außendruck höher ist, als der in der hermetisch abgedichteten Druckkammer herrschende Druck, der zylindrische Faltenbalg des Stellgliedes sich axial staucht und dadurch das Absperrorgan öffnet, wobei der Zeitpunkt des Öffnens beziehungsweise Schließens des Absperrorgans über die Dimensionierung des zylindrischen Faltenbalges sowie Art und Höhe des in die Druckkammer hermetisch eingeschlossenen Mediums vorbestimmt Ist.

Der dehnbare zylindrische Faltenbalg des Stellgliedes wirkt als Vorspannfeder, wenn das Stellglied auf dem Behälter verrastet ist und bei einem Druckverlust z.B. durch ein Leck der hermetisch abgedichteten Druckkammer hält diese Vorspannung das Absperrorgan Druck- und Temperatur unabhängig geschlossen.

Wenn jedes Absperrorgan des Faltenbalges mit einem Ventilsitz zusammenwirkt, der von einer Vertiefung des Behälterunterteils gebildet ist, kann die erfindungsgemäße Ventilanordnung auch zur Flüssigkeitsabfuhr beispielsweise zur Kondensatabfuhr aus dem Sterilisierbehälter Verwendung finden.

Ausgestaltungen der Erfindung ergeben sich aus den Unteransprüchen.

Nachstehend werden Ausführungsbeispiele der Erfindung anhand der Zeichnung veranschaulicht. In der Zeichnung zeigen:
Fig. 1 einen Teilschnitt des Bodens eines für die Dampf-Autoklavierung bestimmten Behälters mit einem FluidAuslass in einer Lage vor dem endgültigen Einsatz;
Fig. 2 eine der Fig. 1 entsprechende Teilansicht des Fluid-Auslasses in Öffnungsstellung;
Fig. 3 eine der Fig. 2 entsprechende Ansicht des Fluid-Auslasses in Schließstellung.

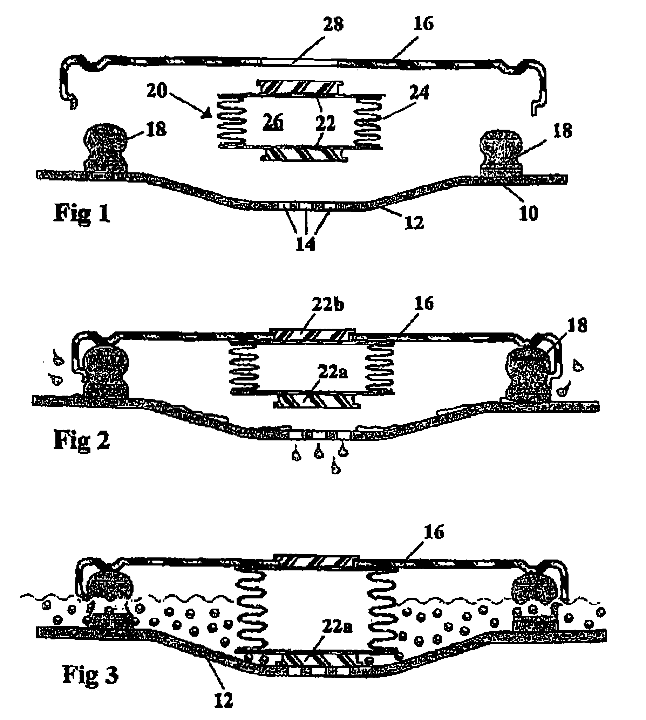

Im Boden 10 eines für die Dampf-Autoklavierung bestimmten Behälters ist in einer Vertiefung 12 ein Auslass 14 angeordnet, der auch als Einlass ausgebildet sein kann. Die Vertiefung 12 im Behälterboden 10 bildet einen Sitz, der den Auslass 14 umschließt. Als Stellglied 20 ist eine Druckkammer vorgesehen, die von einem zylindrischen Faltenbalg 24 aus Edelstahl und zwei endseitig aufgeschweißten Deckeln 22a, 22b gebildet ist. Dadurch wird Innerhalb der Druckkammer ein Gasvolumen (vorzugsweise Luft mit vorbestimmter Temperatur und vorbestimmtem Druck) eingeschlossen, das bei einer vorbestimmten Temperatur einen vorbestimmten Druck aufweist. Die beiden Deckel 22 des Stellgliedes 20 sind identisch und als Rastscheibe ausgebildet. Mittels einer dieser Rastscheiben kann das Stellglied 20 in einer entsprechend bemessenen Ausnehmung 28 einer Trägerkappe 16 eingerastet werden. Die Trägerkappe 16 ist auf den Behälter (Wand oder Boden) aufrastbar, wie aus Fig. 2 ersichtlich, beispielsweise durch Aufrasten auf wenigstens drei im gleichen Winkelabstand auf dem Behälterboden angeordneten Zapfen 18. In der Schaltstellung gemäß Fig. 2 ist der Innendruck des Stellgliedes niedriger als der Außendruck, und der untere, das Stellglied 20 bildende Deckel 22a ist von seinem Sitz abgehoben, denn der in der Kappe 16 eingerastete Deckel 22b trägt das Stellglied 20 und hebt den unteren Deckel von dem Auslass 14 ab, so dass das Fluid aus dem Behälter ausströmen oder in diesen einströmen kann.

Wenn der Druck in der Druckkammer 26 größer wird als der das Stellglied umgebende Außendruck, dann streckt sich der Faltenbalg 24 des Stellgliedes 20, und der Deckel 22a wird auf den Auslass 14 im Behälterboden gedrückt, so dass der Fluid-Auslass abgeschlossen ist.

Die Ausbildung des Stellgliedes 20 als Einzelbauteil, das der Trägerkappe auf einfache Weise entnommen werden kann, hat den Vorteil einer Vereinfachung des Reinigungsvorganges, wobei das Zusammenfügen nach der Reinigung noch dadurch erleichtert wird, dass die beiden Rastdeckel 22 identisch ausgebildet sind, so dass jeder Deckel 22a, 22b entweder als Rastscheibe oder als Abschlusskörper wirken kann.

Gemäß einem weiteren, In der Zeichnung nicht dargestellten Ausführungsbeispiel kann das Stellglied 20 fest oder auswechselbar über einen seiner Deckel 22a, 22b an einer Behälterwand festgelegt werden, wobei der gegenüber liegende Deckel an eine Kappe anstößt oder mit dieser das Abschlussglied eines FluidEin-/Auslasses bildet, wobei eine Öffnung erfolgt, wenn der Innendruck des Stellgliedes größer ist als der Außendruck und ein Schließen bewirkt wird, wenn der Innendruck kleiner ist als der Außendruck. Der Vorteil dieser Anordnung besteht darin also, dass der Einlass dann öffnet, wenn der Innendruck des Stellgliedes größer ist als der Außendruck, so dass durch Kombination von Einlässen und Auslässen ein Behälter geschaffen werden kann, der immer dann offene Durchgangslöcher hat, wenn der Umgebungsdruck vom Normaldruck verschieden ist.

## Patentansprüche

1. Ventil für einen FluidEin- und Auslass (14) eines für die Dampf-Autoklavierung bestimmten Behälters (10), bei dem der Ein- bzw. Auslass (14) von einem Sitz in einer Wandung (12) des Behälters (10) umschlossen ist; wobei:
- das Ventil aus einem Absperrorgan (22a), das mit dem Sitz in Eingriff bringbar ist, einer auf die Wandung (12) des Behälters (10) auswechselbar aufrastbaren Trägerkappe (16) und einem Stellglied (20) besteht, das während einer 10 Autoklavierung abhängig von der Druckdifferenz zur Umgebung des Stellgliedes (20) das Absperrorgan (22a) in die Schließstellung bzw. in die Öffnungsstellung überführt, wobei das Stellglied (20) eine von einem dehnbaren zylindrischen Faltenbalg (24) umschlossene Druckkammer (26) aufweist, die an ihren axialen Enden durch Deckel (22) hermetisch abgeschlossen ist, so dass je nach Druckdifferenz eine axiale Spreizung oder Stauchung entsteht, die auf das Absperrorgan (22a) übertragen wird,
- in der Druckkammer (26) ein Gasvolumen eingeschlossen ist, das bei einer vorbestimmten Temperatur einen vorbestimmten Druck aufweist; die beiden Deckel (22) identisch und als Rastscheibe ausgebildet sind;
- die Rastscheiben (22a, 22b) wahlweise einen Rastkörper (22b) zur Festlegung des Faltenbalges (24) an der Trägerkappe (16) bzw. das Absperrorgan (22a) für den von dem Sitz umschlossenen Ein- bzw. Auslass (14) bilden.

2. Ventil nach Anspruch 1,
**dadurch gekennzeichnet, dass** die Trägerkappe (16) auf die Wandung (12) des Behälters (10) auswechselbar aufrastbar ist.

3. Ventil nach Anspruch 2,
**dadurch gekennzeichnet, dass** der Rastkörper (22a, 22b) in eine Ausnehmung (28) der Trägerkappe (16) auswechselbar einrastbar ist.

4. Ventil nach Anspruch 3, dadurch gekennzeichent, dass die Trägerkappe (16) auf Zapfen_(18) der Wand (12) des Behälters (10) auswechselbar aufrastbar Ist.

5. Ventil nach einem der Ansprüche 1 bis 3, **dadurch gekennzeichnet, dass** der Ventilsitz in einer Vertiefung (12) Im Boden des Behälter (10) angeordnet Ist.

6. Ventil nach einem der vorhergehenden Ansprüche, **dadurch gekennzeichnet, dass** der dehnbare, zylindrische Faltenbalg (24) als Vorspannfeder ausgebildet ist, die das Absperrorgan (22) bei Druckverlust in der Druckkammer (26) unabhängig von Temperatur und Druck geschlossen hält.

7. Ventil nach einem der vorherigen Ansprüche,
**dadurch gekennzeichnet, dass** die als Rastkörper ausgebildeten Deckel (22a, 22b) des Stellgliedes (20) farblich kontrastierend zur Trägerkappe (16) ausgebildet sind.

## Claims

1. Valve for a fluid inlet and outlet (14) of a container (10) intended for steam autoclaving, wherein the inlet or, respectively, outlet (14) is surrounded by a seat in a wall (12) of the container (10); wherein:
- the valve comprises a shut-off valve (22a) which can be made to engage with the seat, an interchangeable support cap (16) to snap on to the wall (12) of the container (10) and an actuator (20) which converts the shut-off valve (22a) into the closed position or, as the case may be, the opening position during autoclaving depending on the pressure difference with the surroundings of the actuator (20), wherein the actuator (20) has a pressure chamber (26) surrounded by an extendable cylindrical bellows (24) which is closed hermetically at each end by covers (22) so that, depending on the pressure difference, axial spreading or compression occurs which is transferred to the shut-off valve (22a),
- a volume of gas is enclosed in the pressure chamber (26) which is under a predetermined pressure at a predetermined temperature; both covers (22) are identical and are formed aslocking discs;
- optionally the locking discs (22a, 22b) form a snap-on body (22b) to secure the bellows (24) to the support cap (16) or, respectively, to form the shut-off valve (22a) for the inlet or outlet (14) surrounded by the seat.

2. Valve in accordance with claim 1,
characterised in thatthe support cap (16) can be snapped interchangeably on to the wall (12) of the container (10).

3. Valve in accordance with claim 2,
characterised in thatthe snap-on body (22a, 22b) can be snapped interchangeably into a recess (28) in the support cap (16).

4. Valvein accordance with claim 3,
characterisedin that the support cap (16)can be snapped interchangeably on to studs (18) on the wall (12) of the container (10).

5. Valve in accordance with one of the claims 1 to 3,
characterised in thatthe valve seat is arranged in a depression (12) in the base of the container (10).

6. Valve in accordance with one of the previous claims,
characterised in thatthe extendable, cylindrical bellows (24) is formed as a preloaded spring which keeps the shut-off valve (22) closed when the pressure is lost in the pressure chamber (26) regardless of temperature and pressure.

7. Valve in accordance with one of the previous claims,
**characterised in that** the covers (22a, 22b) of the actuator (20) formed as snap-on bodies are formed in colours which contrast with that of the support cap (16).

## Revendications

1. Clapet pour entrée et sortie de fluide (14) d'un récipient (10) destiné à l'autoclave par vapeur, dans lequel l'entrée, respectivement, la sortie (14) est entourée par un siège dans une paroi (12) du récipient (10), sachant que :
- le clapet est composé d'un organe de blocage (22a) qui peut être mis en prise avec le siège, d'un bouchon porteur (16) pouvant être encliqueté de façon interchangeable sur la paroi (12) du récipient (10) et d' un membre de réglage (20) qui fait passer l'organe de blocage (22a) de la position fermée respectivement à la position ouverte pendant une autoclave en fonction de la différence de pression par rapport à l'environnement du membre de réglage (20), sachant que le membre de réglage (20) présente une chambre de pression (26) entourée par un soufflet cylindrique extensible (24), qui est fermée hermétiquement par un couvercle (22) sur ses extrémités axiales, de telle sorte que selon la différence de pression, il se produit une extension ou un refoulement, transmise à l'organe de blocage (22a),
- un volume de gaz est pris dans la chambre de pression (26), lequel présente une pression prédéterminée à une température prédéterminée ;
les deux couvercles (22) sont formés à l'identique et en tant que disque à encliquetage ;
- les disques à encliquetage (22a, 22b) présentent au choix un corps d'encliquetage (22b) pour fixer le soufflet (24) sur le bouchon porteur (16), respectivement l'organe de blocage (22a) pour l'entrée, respectivement, la sortie (14) entourée par le siège.

2. Clapet selon la revendication 1,
**caractérisé en ce que** le bouchon porteur (16) peut être encliqueté de façon interchangeable sur la paroi (12) du récipient (10).

3. Clapet selon la revendication 2,
**caractérisé en ce que** le corps d'encliquetage (22a, 22b) peut être encliqueté de façon interchangeable dans un évidement (28) du bouchon porteur (16).

4. Clapet selon la revendication 3,
**caractérisé en ce que** le bouchon porteur (16) peut être encliqueté de façon interchangeable sur des tétons (18) de la paroi (12) du récipient (10).

5. Clapet selon l'une des revendications 1 à 3,
**caractérisé en ce que** le siège de clapet est disposé dans un creux (12) dans le fond du récipient (10).

6. Clapet selon l'une des revendications précédentes,
**caractérisé en ce que** le soufflet cylindrique extensible (24) est formé en tant que ressort de précontrainte qui maintient l'organe de blocage (22a) fermé indépendamment de la température et de la pression en cas de perte de pression dans la chambre de pression (26).

7. Clapet selon l'une des revendications précédentes, **caractérisé en ce que** les couvercles (22a, 22b) du membre de réglage (20) conçus en tant que corps d'encliquetage, sont conçus avec une couleur contrastant avec celle du bouchon porteur (16).
